# EUROPEAN PATENT SPECIFICATION

(11) **EP 2 817 096 B1**
(45) Date of publication and mention of the grant of the patent: **27.03.2019**
(21) Application number: 13752032.6
(22) Date of filing: 13.02.2013
(51) Int. Cl.: B01J 37/08, B01J 37/03, B01J 23/75

(54) **METHODS OF MAKING A CATALYST FOR THE OXIDATIVE DEHYDROGENATION OF OLEFINS**
VERFAHREN ZU IHRER HERSTELLUNG EINES KATALYSATORS ZUR OXIDATIVEN DEHYDRIERUNG VON OLEFINEN
PROCÉDÉS DE FABRICATION D'UN CATALYSEUR POUR LA DÉSHYDROGÉNATION OXYDATIVE DE CATALYSEUR D'OLÉFINES

(30) Priority: 20.02.2012 US 201261600813 P
(43) Date of publication of application: 31.12.2014
(73) Proprietor: Saudi Basic Industries Corporation, 11422 Riyadh (SA)
(72) Inventor: TENNYSON, Reginald Lamont, SugarLand, Texas 77478 (US)
(74) Representative: Stiebe, Lars Magnus
(86) International application number: PCT/US2013/025827
(87) International publication number: WO 2013/126248

(56) References cited:
- EP-A1- 2 873 458
- WO-A1-2012/030891
- CN-A- 1 072 110
- CN-A- 1 074 631
- CN-A- 1 184 705
- CN-A- 102 824 914
- US-A- 3 849 545
- US-A- 4 058 577
- US-A1- 2004 167 013
- US-A1- 2010 112 397
- US-A1- 2011 004 041
- LEE, HOWON ET AL.: 'Preparation of ZnFe204 Catalysts by a Co-precipitation Method Using Aqueous Buffer Solution and Their Catalytic Activity for Oxidative Dehydrogenation of n-Butene to 1,3-Butadiene' CATALYSIS LETTERS vol. 122, 11 December 2007, pages 281 - 286, XP019601266
- JUNG, JI CHUL ET AL.: 'Production of 1,3-Butadiene From C4 Raffinate-3 Through Oxidative Dehydrogenation of n-Butene Over Bismuth Molybdate Catalysts' CATALYSIS SURVEYS FROM ASIA vol. 13, no. IS.2, 28 April 2009, pages 78 - 93, XP019672211
- FAN, YUN ET AL.: 'Destruction of Polychlorinated Aromatic Compounds by Spinel-Type Complex Oxides' ENVIRONMENTAL SCIENCE & TECHNOLOGY vol. 44, no. 8, 24 March 2010, pages 3079 - 3084, XP055082560

## Description

### BACKGROUND

This disclosure relates to catalysts and particularly to catalysts for the oxidative dehydrogenation of organic compounds such as olefins.

The demand for 1,3-butadiene is increasing in petrochemical markets. 1,3-butadiene is produced through a naphtha cracking process, a direct n-butene dehydrogenation reaction, or an oxidative n-butene dehydrogenation reaction, and is then supplied to a petrochemical market. The oxidative n-butene dehydrogenation reaction, which is a reaction for forming 1,3-butadiene and water by reacting n-butene with oxygen, is advantageous in that, since stable water is formed as a product, the reaction is thermodynamically favorable, and the reaction temperature can be lowered.

The oxidative dehydrogenation reaction of n-butene (1-butene, trans-2-butene, cis-2-butene) is a reaction for forming 1,3-butadiene and water by reacting n-butene with oxygen. However, in the oxidative dehydrogenation reaction of n-butene, many side reactions, such as complete oxidation, etc., are predicted because oxygen is used as a reactant. For this reason, a catalyst which can suppress these side reactions to the highest degree and which has high selectivity to 1,3 butadiene is highly desirable.

Many patents are devoted to improving selectivity and yield. It is understood that in the oxidative dehydrogenation reaction of n-butene, when the zinc ferrite catalyst is substituted with other metals or the mixed-phase catalyst is used, high activity can be obtained in the production of 1,3-butadiene, compared to when a conventional zinc ferrite catalyst is used, but there is a problem in that it is difficult to synthesize and to reproduce the catalyst, and thus it is difficult to commercialize the catalyst.

Methods for preparing hydrogenation catalysts are known from US 4 058 577 A, CN 1 184 705 A, CN 1 074 631 A and Catalysis letters (2008) Volume 122, pages 281-286

Hence, there is a continual need for dehydrogenation catalysts that retain or improve yield, conversion, and/or selectivity, while forming a reproducible catalyst and/or reducing the cost of the catalyst (e.g., production costs by reducing the amount of starting zinc material and/or recovering a significant portion of the starting zinc material).

### BRIEF DESCRIPTION

In an embodiment, there is a method of making a dehydrogenation catalyst comprising: combining a zinc precursor, an iron precursor, a cobalt precursor, a magnesium precursor, optionally a calcium precursor, and optionally a M in water to form a mixture; adding base to the mixture to form a slurry having a pH of 7 to 8.5; aging the slurry at a temperature of greater than or equal to 40°C while agitating; filtering a precipitate from the aged slurry to collect a catalyst precursor; drying and calcining the catalyst precursor to form the catalyst; wherein the catalyst has the formula (I)

FeZnₐCo_{b}Mg_{c}Ca_{d}ClₑM_{f}Oₓ (I)

wherein the amounts are in mole ratios relative to 1 mole of iron, "a" is 0.07 to 0.7 moles; "b" is 0.01 to 0.20 moles; "c" is less than or equal to 0.40 moles; "d" is less than or equal to 0.40 moles; "e" is less than or equal to 0.10 moles; "f" is less than or equal to 0.20 moles; and "x" is a number depending on the relative amount and valence of the elements different from oxygen in Formula (I), wherein greater than about 88% of the initial zinc is recovered, and wherein the mole ratio of the initial zinc to the initial iron is less than or equal to 0.35. M can be selected from cobalt (Co), magnesium (Mg), calcium (Ca), silver (Ag), aluminum (Al), cerium (Ce), cesium (Cs), copper (Cu), potassium (K), lanthanum (La), lithium (Li), manganese (Mn), molybdenum (Mo), sodium (Na), nickel (Ni), phosphorus (P), palladium (Pd), platinum (Pt), ruthenium (Ru), silicon (Si), vanadium (V), tungsten (W), yttrium (Y), and combinations comprising at least one of the foregoing.

In another embodiment, there is a method of making a dehydrogenation catalyst comprising: combining a zinc precursor, an iron precursor, a cobalt precursor, a magnesium precursor, optionally a calcium precursor, and optionally a M in water to form a mixture; adding base to the mixture to form a slurry having a pH of 7 to 8.5; aging the slurry at a temperature of greater than or equal to 40°C while agitating; filtering a precipitate from the aged slurry to collect a catalyst precursor; drying and calcining the catalyst precursor to form the catalyst; wherein the catalyst has the formula (I)

FeZnₐCo_{b}Mg_{c}Ca_{d}ClₑM_{f}Oₓ (I)

wherein the amounts are in mole ratios relative to 1 mole of iron, "a" is 0.07 to 0.7 moles; "b" is 0.01 to 0.20 moles; "c" is less than or equal to 0.40 moles; "d" is less than or equal to 0.40 moles; "e" is less than or equal to 0.10 moles; "f" is less than or equal to 0.20 moles; and "x" is a number depending on the relative amount and valence of the elements different from oxygen in Formula (I), and wherein if the pH is greater than 8.1 then the aging time is greater than or equal to 90 minutes and/or the aging temperature is greater than or equal to 75°C, and wherein the mole ratio of the initial zinc to the initial iron is less than or equal to 0.35. M can be selected from cobalt (Co), magnesium (Mg), calcium (Ca), silver (Ag), aluminum (Al), cerium (Ce), cesium (Cs), copper (Cu), potassium (K), lanthanum (La), lithium (Li), manganese (Mn), molybdenum (Mo), sodium (Na), nickel (Ni), phosphorus (P), palladium (Pd), platinum (Pt), ruthenium (Ru), silicon (Si), vanadium (V).

In another embodiment, there is a method of making a dehydrogenation catalyst, by combining yellow iron (III) oxide monohydrate with water and an acid to form a slurry, agitating the slurry at a temperature of 25°C to 80°C for a period of greater than or equal to 1 hour; adding the zinc (Zn) precursor, optionally the cobalt (Co) precursor, optionally the magnesium (Mg) precursor, and optionally MX to form a slurry; spray drying to form the catalyst precursor; and calcining the catalyst precursor to form the catalyst. M can be selected from calcium (Ca), silver (Ag), aluminum (Al), cerium (Ce), cesium (Cs), copper (Cu), potassium (K), lanthanum (La), lithium (Li), manganese (Mn), molybdenum (Mo), sodium (Na), nickel (Ni), phosphorus (P), palladium (Pd), platinum (Pt), ruthenium (Ru), silicon (Si), vanadium (V), tungsten (W), yttrium (Y), and combinations comprising at least one of the foregoing, and wherein X is an oxide, nitrate, carbonate, halide, or a hydrate thereof. The catalyst has the formula:

FeZnₐCo_{b}Mg_{c}Ca_{d}ClₑM_{f}Oₓ (I)

wherein the amounts are in mole ratios relative to 1 mole of iron, "a" is 0.07 to 0.7 moles; "b" is 0.01 to 0.20 moles; "c" is less than or equal to 0.40 moles; "d" is less than or equal to 0.40 moles; "e" is less than or equal to 0.10 moles; "f" is less than or equal to 0.20 moles; and "x" is a number depending on the relative amount and valence of the elements different from oxygen in Formula (I).

In another embodiment, a method of making a dehydrogenation catalyst can comprise: combining a zinc precursor, an iron precursor, and optionally an MX, to form a slurry; agitating the slurry for greater than or equal to 2 hours at a temperature of greater than or equal to 25°C; spray drying the slurry to form the catalyst precursor; and calcining the catalyst precursor to form the catalyst. M can be selected from cobalt (Co), magnesium (Mg), calcium (Ca), silver (Ag), aluminum (Al), cerium (Ce), cesium (Cs), copper (Cu), potassium (K), lanthanum (La), lithium (Li), manganese (Mn), molybdenum (Mo), sodium (Na), nickel (Ni), phosphorus (P), palladium (Pd), platinum (Pt), ruthenium (Ru), silicon (Si), vanadium (V), tungsten (W), yttrium (Y), and combinations comprising at least one of the foregoing, and wherein X is an oxide, nitrate, carbonate, halide, or a hydrate thereof. The catalyst has the formula (I)

FeZnₐCo_{b}Mg_{c}Ca_{d}ClₑM_{f}Oₓ (I)

wherein the amounts are in mole ratios relative to 1 mole of iron, "a" is 0.07 to 0.7 moles; "b" is 0.01 to 0.20 moles; "c" is less than or equal to 0.40 moles; "d" is less than or equal to 0.40 moles; "e" is less than or equal to 0.10 moles; "f" is less than or equal to 0.20 moles; and "x" is a number depending on the relative amount and valence of the elements different from oxygen in Formula (I).

The above described and other features are exemplified by the following figures and detailed description.

### DETAILED DESCRIPTION

It was discovered that a catalyst with good reactivity (e.g., conversion of n-butene of greater than or equal to 75%) and/or selectivity to 1,3 butadiene (e.g., greater than or equal to 91%), e.g., at an oxygen conversion level of greater than or equal to 85%, can be made with a reduced amount of zinc (where, for example, the mole ratio of the initial zinc to the initial iron can be less than or equal to 0.35, specifically 0.20-0.35, more specifically 0.20-0.30) and/or reduced amount of base compared to conventional levels. In the present process the amount of base is sufficient to attain a pH of less than or equal to 8.5 specifically a pH of 7 to 8.5, more specifically, a pH of 7 to 8.1, even more specifically, a pH of 7 to 7.75, yet more specifically, a pH of 7 to 7.5, and still more specifically, 7.2 to 7.5; alternatively, the amount of base is sufficient to attain a pH of 8.2 to 8.5. Not to be limited to theory, employing less zinc allows the production of the catalyst at a lower pH while attaining a similar performance as compared to forming the catalyst with greater amounts of zinc at a pH of 8.2 to 8.5. In other words, by employing a lower pH, the starting amounts of zinc can be reduced from conventional levels by, for example, about 35% and a high zinc recovery of greater than about 88%, specifically of greater than or equal to about 90% is achieved, more specifically of greater than or equal to about 92% is achieved, where less than or equal to 60% of zinc recovery is not acceptable. Unexpectedly, it was discovered that by reducing the pH, the amount of zinc needed to obtain a catalyst having similar or better selectivity and conversion as catalyst formed at a higher pH, was less and the amount of zinc recovered was more. It was further found that when a pH of 8.2 to 8.5 was used, that high zinc recovery could be obtained by either increasing the digest time, for example to greater than 90 minutes and/or increasing the digest temperature, for example to greater than or equal to 75°C, specifically greater than or equal to 90°C (e.g., greater temperatures can be used if higher pressure is employed). The present catalyst has a n-butene to butadiene conversion of greater than or equal to 75%, specifically, greater than or equal to 77% at a temperature of 320°C to 400°C and a pressure of 0 to 30 psig (101 to 308 kPa). This conversion can be maintained for greater than or equal to 40 hours (hrs). The present catalyst has a selectivity for 1,3 butadiene of greater than or equal to 91%, specifically, greater than or equal to 92%.

The catalyst formed herein can comprise iron (Fe), zinc (Zn), cobalt (Co), magnesium (Mg), calcium (Ca), and chloride (Cl), as set forth in the following Formula (I):

FeZnₐCo_{b}Mg_{c}Ca_{d}ClₑM_{f}Oₓ (I)

wherein the amounts are in mole ratios relative to 1 mole of iron:
"a" is 0.07 to 0.7 moles (e.g., 0.15 to 0.35 moles);
"b" is 0.01 to 0.20 moles, (e.g., 0.02 to 0.08 moles);
"c" is less than or equal to 0.40 (e.g., 0.001 to 0.40 moles, specifically, 0.001 to 0.15 moles);
"d" is less than or equal to 0.40 moles (e.g., 0.0 to 0.40 moles, specifically, 0.001 to 0.08 moles);
"e" is less than or equal to 0.10 moles (e.g., 0.0 to 0.10 moles, specifically, 0.0001 to 0.10 moles, more specifically, 0.001 to 0.05 moles);
"f" is less than or equal to 0.20 moles (e.g., 0.0 to 0.20 moles, specifically, 0.0001 to 0.20 moles);
"x" is a number depending on the relative amount and valence of the elements different from oxygen in Formula (I); and
M is silver (Ag), aluminum (Al), cerium (Ce), cesium (Cs), copper (Cu), potassium (K), lanthanum (La), lithium (Li), manganese (Mn), molybdenum (Mo), sodium (Na), nickel (Ni), phosphorus (P), palladium (Pd), platinum (Pt), ruthenium (Ru), silicon (Si), vanadium (V), tungsten (W), yttrium (Y), as well as combinations comprising at least one of the foregoing.

The catalyst can be produced by combining zinc precursor(s), iron precursor(s), cobalt precursor(s), magnesium precursor(s), calcium precursor(s), and M precursor(s) in water to form a mixture. Examples of possible precursors include soluble: oxide precursors, nitrate precursors, carbonate precursors, halide precursors, and combinations comprising at least one of the foregoing precursors. Some specific precursors include: nitrate, nitrate nonahydrate, nitrate hexahydrate, oxide, oxide monohydrate, carbonate, carbonate hydrate, chloride, as well as combinations comprising at least one of the foregoing. The amounts of the various precursors are chosen to attain the above mole ratios relative to 1 mole of iron.

A base is added to the mixture to attain a slurry having a pH of 7 to less than 8.5, specifically to a pH of 8.2 to 8.5. Alternatively, a slurry having a pH of 7 to 8.1, specifically to a pH of 7 to 7.75, and more specifically, to a pH of 7.2 to 7.5. Some possible alkaline solutions include sodium hydroxide and/or ammonium hydroxide, however ammonium hydroxide is desirable in that it tends to leave less residue than, for example, sodium hydroxide after calcination.

Once the desired pH has been attained, the slurry is then aged (also referred to as "digest"). During the aging process, the particles can agglomerate and their resultant size can correspondingly increase. Desirably, this process is performed at elevated temperatures to facilitate water removal from the particles and resulting in particle agglomeration e.g., at a temperature of greater than or equal to 40°C, specifically, at 40°C to 90°C, more specifically at 40°C to 75°C. The duration of the aging process is dependent upon the temperature at which the aging occurs. For example, if the aging temperature is 40°C, aging of the slurry for about 2 hours may be adequate, but if the aging temperature is increased to 75°C, the aging process can be completed in less than 2 hours. Agitating (e.g., stirring) may or may not be employed during the aging process.

After aging, the precipitate can be filtered and optionally washed (e.g., with water such as deionized water) to collect the catalyst precursor e.g., in the form of a gel-like cake. Filtration can be performed at the temperature of the slurry. Optionally the slurry can be filtered at a temperature of 40°C to 90°C, specifically at a temperature of 40°C to 75°C.

The catalyst precursor is then dried and calcined to form the catalyst which can be sized and used for n-butene oxidation. The catalyst precursor can be dried passively and/or actively, e.g., can be allowed to dry (e.g., at room temperature), can be heated to facilitate drying, and/or can be spray dried.

Alternatively, the catalyst can be prepared by combining a zinc precursor, an iron precursor, MX, and acid, to form a slurry. Advantageously, forming the slurry can comprise combining the iron precursor with the acid to form an acid mixture, and agitating the acid mixture, e.g., for a period of greater than or equal to 0.5 hours, specifically, 1 to 5 hours. The zinc precursor and any MX precursors can then be combined with the stirred acid mixture to form the slurry. The slurry can be stirred to form a thickened slurry, e.g., at a temperature of 25°C to 80°C, specifically, 25°C to 30°C. The slurry can optionally be stirred for a period of time, e.g., for greater than or equal to 1 hour. The thickened slurry is then spray dried to form the catalyst precursor and then calcined.

Such methods are defined in appended claims 8 and 9.

Once dried, the catalyst can be calcined at a temperature of 400°C to 650°C, e.g., for a period of up to 15 hours or so (e.g., 1 to 15 hrs). For example, the precursor can be calcined at 650°C for 10 hours.

The catalyst can be sized for the particular application, e.g., reactor size. The sizing can be accomplished by any known method, with the particular method dependent upon the manner in which the catalyst was prepared. For example, if the calcined catalyst is relatively large, the catalyst can be ground into smaller particles before screening. Likewise, if the catalyst precursor is prepared so as to have a very small particle size, e.g., via a drying method such as spray drying, the catalyst can be pressed first, optionally ground, and then screened to form the desired particle size. One skilled in the art understands that the optimum sized catalyst particle is different depending upon the reactor employed whereby bigger reactors generally employ larger particles. In one embodiment, the catalyst can be screened using a 20 - 50 mesh size (e.g., 297 to 841 micrometers).

Optionally, the catalyst precursor can be formed by combining the metals, e.g., in the form of oxides, nitrates, carbonates, or halides, optionally with a dispersant and optionally with promoters to form a slurry. For example, zinc precursor, iron precursor, and cobalt precursor, magnesium precursor, MX, wherein M is defined as above and X is an oxide, nitrate, carbonate, halide, or hydrates thereof, and an acid dispersant, are combined to form a slurry. Possible acid dispersants include hydrochloric acid (HCl), ferrous chloride (FeCl₃), nitric acid (HNO₃), phosphoric acid (H₃PO₄), and/or zinc chloride (ZnCl₂). In an embodiment, the dispersant is hydrochloric acid. In this embodiment, the hydrochloric acid can be present such that the iron to chloride ratio is 1:0.00 to 1:0.35 (e.g., 1:0.001 to 1:0.35), specifically, 1:(greater than 0.00) to 1:0.23 (e.g., 1:0.01 to 1:0.23). Desirably, the amount of chloride is limited such that there is chloride at less than or equal to 0.23 chloride per 1 iron, such that the formation of zinc chloride (ZnCl₂), which has a boiling point of about 756°C, is prevented or minimized, as the presence of zinc in the form of ZnCl₂ provides a mechanism for some of the zinc to leave the catalyst during the calcination step. In an embodiment, the iron precursor is yellow iron (III) oxide monohydrate.

Although the metals can all be mixed together in a single step, it is desirable to combine the iron oxide with the acid to form an acid mixture. The acid mixture can be agitated for a period of greater than or equal to 0.5 hours, specifically, greater than or equal to 1.0 hours to form a stirred acid mixture. The zinc precursor, magnesium precursor, the calcium precursor, and any other MX precursors can then be added to the acid mixture containing the iron oxide.

The slurry can optionally be stirred at a temperature of 25°C to 80°C for a period of greater than or equal to 1 hour, specifically, 1 hour to 5 hours, forming a thickened slurry. For example, the mixture is stirred at 50°C for 2 hours.

The thickened slurry can then be dried to form the catalyst precursor. Optionally, the thickened slurry can be spray dried, e.g., without filtration and/or without washing. This process produces minimal waste, essentially only fines trapped in the spray dryer filter. The catalyst precursor can then be calcined and/or sized as described above.

The catalyst formed by any of the above methods can be used alone or can be deposited on a support carrier by various methods. For example, an aqueous mixture (e.g., solution or dispersion) of the catalyst can be prepared and mixed with the support e.g., until the active ingredients are coated on the support. Examples of support materials include alumina, silica gel, silica-alumina, silicon carbide, pumice, firebrick, kieselguhr, quartz, and combinations comprising at least one of the foregoing. The support can be in various forms such as shaped bodies including tablets, pellets, particulates, cylinders, hollow cylinders, rings, spheres, strands, wagon wheels, saddles, extrudates, "trilobes", "tristars", bodies having at least one notch on the exterior, as well as combinations comprising at least one of the foregoing.

The disclosed catalyst can be used for the oxidative dehydrogenation of organic compounds such as olefins by contacting them, in for example a reactor (e.g., pressure vessel or fluidized bed), with a gas mixture comprising the olefin and oxygen. Without being bound by theory, it is believed that the oxidative dehydrogenation reaction occurs via the catalyst through the process of sorption (absorption and/or adsorption) of the reactant such as n-butene to the catalyst, reacting oxygen existing in the lattice of the catalyst with two hydrogen atoms of the n-butene adsorbed on the catalyst to produce 1,3-butadiene and water, and filling the vacant oxygen sites of the lattice of the catalyst with the reactant oxygen.

In an embodiment, contact between the catalyst and the gas mixture occurs by passing the gas mixture through the interstices in a reactor such as a fixed bed reactor, which ensures intimate contact between the gas mixture and the catalyst. In embodiments wherein the gas cannot pass through the catalyst, the gas mixture may be passed over the catalyst surface. During contact, the catalyst and the gas mixture can be maintained at a temperature of 320°C to 430°C, thus the reaction temperature is typically 400°C or less. The reaction pressure during contacting can be 0 to 30 pounds per square inch gauge (psig) (e.g., 101 to 308 kPa). Generally, the gas mixture that contacts the catalyst comprises the olefin to be dehydrogenated and oxygen or an oxygen source in the reactor. The gas mixture may also include diluents such as steam, nitrogen, argon, and/or carbon dioxide. Optionally, the gas mixture can include butylenes, ethylene, propylene, or a combination comprising at least one of the foregoing. Typically the gas mixture that is contacted with the catalyst has an oxygen content of 3 to 10 percent by volume (vol%) and an olefin content of 4 to 15 vol%. Steam, which can be effective in removing the reaction heat caused by an oxidative dehydrogenation reaction to improve selectivity for 1,3-butadiene, may be supplied to the reactor. In an embodiment, steam is provided by adding liquid water to the reactor, which vaporizes to steam.

The following examples are intended to further explain the present catalyst and methods for making the catalyst. These methods are exemplary, and not limiting.

### EXAMPLES

| Table 1: Components | |
|---|---|
| Component | Source |
| Iron nitrate nonahydrate [Fe(NO₃)₃*9H₂O] | Aldrich |
| Zinc nitrate hexahydrate | Aldrich |
| Cobalt(II) nitrate hexahydrate 98% | Aldrich |
| Magnesium nitrate hexahydrate | Alfa Aesar |
| Calcium nitrate tetrahydrate | Aldrich |
| Sesbania | SeedLand |
| Ammonium hydroxide solution 28% | Aldrich |
| Iron (III) oxide monohydrate [Fe(NO₃)₃*H₂O] | Strem |
| Iron (III) oxide < 5 µm powder [Fe₂O₃] | Aldrich |
| Iron (III) oxide (Geothite) [Fe(OH)O] | Aldrich |
| Yellow iron (III) oxide monohydrate [Fe₂O₃*H₂O] | Stem |
| Iron nitrate | Aldrich |

### Example 1: Formation of catalyst at a low pH

Table 1 provides a list of the components and the source. It is clearly understood that this information is included for convenience and completeness. Other sources of the various components can be substituted for those listed in Table 1.

The components as listed in Table 2, iron nitrate nonahydrate, zinc nitrate hexahydrate, cobalt(II) nitrate hexahydrate 98%, magnesium nitrate hexahydrate, and calcium nitrate tetrahydrate were combined with 750 milliliters (mL) deionized water. To this mixture 0.76 g of ground sesbania, was added. The mixture was stirred. Diluted ammonium hydroxide solution (about 14 wt%) was added until the slurry reached a pH of 5.6. The resulting slurry was aged at a temperature of 75°C for 30 minutes. The slurry was cooled to 40°C, then filtered. The resulting gel-like cake was washed with deionized water to form the catalyst precursor. The catalyst precursor was then dried at a temperature of 120°C for 1 hour and calcined at a temperature of 650°C for 10 hours. After calcination, 35 g of catalyst were recovered. The resulting catalyst was screened and the 20/50 mesh fraction was used in n-butene oxidative dehydrogenation.

| Table 2: Starting Materials for Examples 1-9 using the standard amount of zinc nitrate hexahydrate | |
|---|---|
| Component | Weight (g) |
| Iron nitrate nonahydrate | 161.60 |
| Zinc nitrate hexahydrate | 53.31 |
| Cobalt(II) nitrate hexahydrate 98% | 4.89 |
| Magnesium nitrate hexahydrate | 0.51 |
| Calcium nitrate tetrahydrate | 1.79 |

### Examples 2-9: Formation of catalyst at pH values of 7.6 and greater

Catalysts 2-9 were prepared using the same procedure described Example 1, except that the pH of the slurry at the end of ammonium hydroxide addition was varied is as indicated in Table 3. The amount and the properties of the recovered catalyst are given in Table 3. The resulting catalyst was screened and the 20/50 mesh fraction was used in n-butene oxidative dehydrogenation.

| Table 3: Catalyst recovery and metal content of final catalyst using the standard amount of zinc starting material and the effect of slurry pH. | | | | | | | | |
|---|---|---|---|---|---|---|---|---|
| Catalyst Example | pH | Digest temp/time | Filtration temp. °C | Catalyst wt (g) after 650°C Calcination | Metal in final catalyst (wt%) | | Metal recovery (%) | |
| | | | | | Fe | Zn | Fe | Zn |
| 1 | 5.6 | 75°C/ 30 min | 40 | 35 | 63.3 | 7.2 | 99 | 21 |
| 2 | 7.6 | 75°C/ 30 min | 40 | 46 | 49.0 | 22.5 | 101 | 88 |
| 3 | 8.0 | 75°C/ 30 min | 40 | 45 | 50.3 | 20.8 | 101 | 80 |
| 4 | 8.3 | 75°C/ 30 min | 40 | 44 | 52.2 | 18.3 | 100 | 75 |
| 5 | 8.5 | 75°C/ 30 min | 40 | 42 | 52.7 | 16.8 | 98 | 60 |
| 6 | 8.7 | 75°C/ 30 min | 40 | 42 | 53.3 | 16.8 | 99 | 60 |
| 7 | 9.0 | 75°C/ 30 min | 40 | 41 | 54.5 | 14.7 | 99 | 51 |
| 8 | 9.3 | 75°C/ 30 min | 40 | 39 | 56.4 | 12.0 | 99 | 40 |
| 9 | 9.5 | 75°C/ 30 min | 40 | 39 | 57.7 | 10.9 | 100 | 36 |

| | | | | | | | | |
|---|---|---|---|---|---|---|---|---|
| *The Initial Zn/Fe mole ratio for Catalysts 1 - 9 was 0.45. | | | | | | | | |

The results in Table 3 demonstrate that when the standard amount of zinc (e.g., a zinc:iron molar ratio of greater than or equal to 0.4 moles) is initially added and the slurry pH is adjusted to a pH of 5.6, only 21% of the zinc is recovered. Likewise, when the pH is adjusted to a pH of 7.6 or greater, the amount of zinc in the final catalyst declined steadily from 20.8 wt% at pH 8.0 to 10.9 wt% at pH 9.5. Consequently, the recovered amount of zinc in the final catalyst when compared to the starting amount of zinc used in the preparation of the catalyst declined from 88% to 36% when the pH was increased from pH 7.6 to pH 9.5. It is evident from Table 3 that the optimum pH for the recovery of zinc is at a pH of about 7.6.

### Examples 10-18: Formation of catalyst at pH values between 7.2 and 8.5

Catalysts 10-18 were prepared using the same procedure described in Example 1, except that the amount of zinc nitrate hexahydrate used was reduced by about 35% to 34.53 g (approximately 65% of the zinc nitrate hexahydrate used in Examples 1 - 9) and the pH of the slurry at the end of ammonium hydroxide addition is as indicated in Table 4. The amount and the properties of the recovered catalyst are given in Table 4.

| Table 4: Catalyst recovery and metal content of catalyst prepared with reduced amount of zinc starting material and the effect of slurry pH. | | | | | | | | |
|---|---|---|---|---|---|---|---|---|
| Ex. | pH | Digest temp/time | Filtration temp. (°C) | Catalyst wt (g) after 650°C Calcination | Metal in final catalyst (wt%) | | Metal recovery (%) | |
| | | | | | Fe | Zn | Fe | Zn |
| 10 | 7.5 | 75°C/ 30 min | 40 | 41.6 | 53.3 | 17.0 | 99 | 93 |
| 11 | 7.2 | 75°C/ 30 min | 75 | 41.6 | 53.1 | 17.0 | 99 | 93 |
| 12 | 7.2 | 75°C/ 30 min | 75 | 41.0 | 53.1 | 17.0 | 97 | 92 |
| 13 | 8.1 | 75°C/ 30 min | 75 | 41.0 | 53.8 | 16.6 | 99 | 90 |
| 14 | 8.5 | 75°C/ 30 min | 75 | 40.2 | 54.8 | 14.9 | 99 | 79 |
| 15 | 8.5 | 75°C/ 90 min | 75 | 40.8 | 54.2 | 16.3 | 99 | 88 |
| 16 | 8.5 | 75°C/ 180 min | 75 | 40.9 | 53.4 | 16.9 | 98 | 91 |
| 17 | 8.5 | 75°C/ 240 min | 75 | 41.3 | 54.2 | 16.9 | 100 | 92 |
| 18 | 8.5 | 90°C/ 120 min | 75 | 42.2 | 53.2 | 16.7 | 100 | 93 |

| | | | | | | | | |
|---|---|---|---|---|---|---|---|---|
| *The Initial Zn/Fe mole ratio for Catalysts 10 - 18 was 0.29. | | | | | | | | |

Table 4 demonstrates that when the pH is adjusted to 7.2 and 7.6, the amount of zinc recovery is greater than 85%, actually, greater than 88%, and generally greater than 90% and consistently resulted in catalysts with approximately 17.0 wt% zinc in the final catalyst. This is especially surprising as these experiments were performed at a reduced amount of zinc starting material. Specifically, Example 10 resulted in a 93% zinc recovery when the slurry pH was adjusted to 7.5 and Examples 11 and 12 resulted in a 93 and 92% zinc recovery, respectively, when the slurry pH was adjusted to 7.2.

Table 4 shows that at a higher pH of 8.5 however, only 79-88% of the zinc is recovered under similar conditions (30 and 90 minutes digest time for Examples 14 and 15, respectively). Table 4 further shows that to recover more of the zinc at pH 8.5, digest time had to be extended to more than 90 minutes (180 and 240 minutes for Examples 16 and 17, respectively). The table also shows that the temperature can alternatively (or in addition) be increased to recover the desired zinc. For example, the digest time can be greater than 90 minutes, with a higher temperature, e.g., 90°C, with 93% of the zinc recovered at a digest time of 120 minutes (Example 18). This allows a much lower initial zinc amount than other processes, while attaining a final zinc amount that is similar to the prior processes; thereby reducing zinc costs.

### Examples 19-53: Catalyst performance for oxidative dehydrogenation of n-butene

The following conditions were used to test the performance of all of the catalysts in these examples. Conditions used for oxidative dehydrogenation of n-butene were as follows. The catalyst testing station was equipped with a preheated evaporator set at 310°C. The reactor tube was 12 inches (30.5 centimeters (cm)), 316 SS (stainless steel) with ½ inch (1.27 cm) OD and 0.065 inch (0.17 cm) wall thickness. The reactor was heated using a heating jacket equipped with a 12 inch (30.5 cm) heated zone. The temperature of the reactor tube was controlled by a temperature controller with a thermocouple attached to the metal sleeves that clamps to the reactor tube. The reactor tube was equipped with an internal thermocouple housed in a thermowell and was located in the center of the catalyst bed. 6.4 grams (g) of catalyst was mixed with enough inert diluent such as quartz chips to form a constant catalyst bed volume of 10 cubic centimeters (cm³). Stainless steel spacers were used to center the catalyst bed in the reactor tube, which were held in place with small plugs of quartz wool. A gas mixture with the following composition was preheated to 310°C and fed to the reactor: n-butene:oxygen:steam:methane, 1:0.65:11.9:1. The total volume of the feed is approximately 450 SCCM (standard cubic centimeters per minute). The methane was inert under the current reaction conditions and was used as an internal standard for conversion and selectivity calculations.

The temperature in the reactor was controlled using the heating jacket. The heating jacket temperature set point at the skin of the reactor (RX SPT Temp (°C)) was set between 330 and 400°C. The catalysts were usually screened for n-butene oxidative dehydrogenation to butadiene for 48-165 hours (hrs) to determine their performance. Occasionally, catalysts were tested for longer periods of time to assess the catalyst stability under reaction conditions. In the first 24 hours of the catalyst performance test, the jacket temperature was set at a temperature between 330 and 400°C such that 85% of the oxygen in the feed stream was consumed in the reactor. After the first 24 hours, the set temperature of the reactor jacket was typically increased such that 95% of the oxygen in the feed stream was consumed in the reactor. The RX SPT Temp in °C for each catalyst performance test is listed in Table 5 and Table 6. It is important to note that the composition of the feed was oxygen limiting (n-butene to O₂ ratio of 1:0.65), therefore the carbon conversion can be limited by the reactor temperature as well as the available amount of oxygen in the reaction mixture.

The performance of Catalysts 1-9 prepared with the standard amount of zinc is given in Table 5. The performance of Catalysts 10-18 prepared with reduced starting zinc amount is given in Table 6.

| Table 5: Performance of Catalysts 1-9 in n-butene Oxidative dehydrogenation to butadiene. | | | | | | | |
|---|---|---|---|---|---|---|---|
| Performance Example | Catalyst Example | pH | Time (hrs) | RX SPT Temp (°C) | % n-butene Conversion | % O₂ Conversion | % Selectivity to C₄H₆ |
| 19 | 1 | 5.6 | 50 | 379 | 69.1 | 95.3 | 89.5 |
| 20 | 2 | 7.6 | 51 | 374 | 73.2 | 95.2 | 90.5 |
| 21 | 2 | 7.6 | 126 | 388 | 66.0 | 89.9 | 89.8 |
| 22 | 3 | 8.0 | 51 | 372 | 76.7 | 93.9 | 91.5 |
| 23 | 3 | 8.0 | 91 | 372 | 72.9 | 89.7 | 91.6 |
| 24 | 4 | 8.3 | 48 | 361 | 76.0 | 95.7 | 92.7 |
| 25 | 4 | 8.3 | 159 | 363 | 75.2 | 95.0 | 92.6 |
| 26 | 5 | 8.5 | 51 | 356 | 77.0 | 96.5 | 92.0 |
| 27 | 5 | 8.5 | 134 | 357 | 75.7 | 95.1 | 92.1 |
| 28 | 6 | 8.7 | 49 | 357 | 78.7 | 96.2 | 92.4 |
| 29 | 6 | 8.7 | 160 | 359 | 77.7 | 96.3 | 92.3 |
| 30 | 7 | 9.0 | 49 | 343 | 70.7 | 95.2 | 90.1 |
| 31 | 7 | 9.0 | 209 | 347 | 69.1 | 95.6 | 90.0 |
| 32 | 8 | 9.3 | 50 | 338 | 60.3 | 94.5 | 86.1 |
| 33 | 8 | 9.3 | 210 | 344 | 61.6 | 97.2 | 86.1 |
| 34 | 9 | 9.5 | 50 | 338 | 52.9 | 94.5 | 83.5 |
| 35 | 9 | 9.5 | 91 | 338 | 51.8 | 92.0 | 83.2 |

Examining the catalyst performance data presented in Table 5, we conclude that catalyst produced at the following pH range (pH 8.0 to pH 8.7) has optimum performance, where the n-butene exceeds 75% and the selectivity of butadiene exceeds 91%. These catalysts have a zinc content that ranges from 16.8 to 20.8% as evident from Table 3.

The performance of Catalysts 10-18 prepared with a reduced amount of zinc in n-butene oxidative dehydrogenation is presented in Table 6 (Examples 36-53).

| Table 6: Performance of Catalysts 10-18 in n-butene oxidative dehydrogenation to butadiene. Catalyst prepared with reduced starting amount of zinc at pH of 7.2-7.5 | | | | | | | |
|---|---|---|---|---|---|---|---|
| Performance Example | Catalyst Example | pH | Time (hrs) | RX SPT Temp (°C) | % n-butene Conversion | % O₂ Conversion | % Selectivity to C₄H₆ |
| 36 | 10 | 7.5 | 53 | 366 | 77.7 | 96.2 | 92.3 |
| 37 | 10 | 7.5 | 157 | 366 | 76.5 | 95.2 | 92.5 |
| 38 | 11 | 7.2 | 51 | 365 | 77.2 | 95.6 | 92.6 |
| 39 | 11 | 7.2 | 136 | 365 | 76.8 | 95.2 | 92.8 |
| 40 | 12 | 7.2 | 51 | 363 | 78.4 | 95.8 | 91.4 |
| 41 | 12 | 7.2 | 137 | 363 | 77.6 | 95.0 | 91.5 |
| 42 | 13 | 8.1 | 50 | 350 | 78.5 | 94.5 | 93.6 |
| 43 | 13 | 8.5 | 144 | 351 | 79.8 | 96.6 | 93.5 |
| 44 | 14 | 8.5 | 51 | 348 | 78.6 | 95.4 | 92.3 |
| 45 | 14 | 8.5 | 148 | 348 | 77.2 | 94.1 | 93.2 |
| 46 | 15 | 8.5 | 56 | 354 | 80.9 | 96.3 | 91.5 |
| 47 | 15 | 8.5 | 154 | 352 | 80.3 | 95.9 | 91.1 |
| 48 | 16 | 8.5 | 55 | 353 | 79.8 | 96.9 | 92.8 |
| 49 | 16 | 8.5 | 155 | 351 | 78.7 | 95.7 | 92.6 |
| 50 | 17 | 8.5 | 53 | 357 | 78.6 | 96.5 | 92.5 |
| 51 | 17 | 8.5 | 149 | 357 | 79.5 | 97.8 | 92.5 |
| 52 | 18 | 8.5 | 54 | 352 | 78.3 | 95.4 | 93.6 |
| 53 | 18 | 8.5 | 154 | 352 | 79.1 | 95.0 | 93.7 |

Table 6 demonstrates that n-butene conversion for Catalysts 10-18 is greater than 76% and the selectivity to butadiene is greater than 91%. The performance in n-butene oxidation of Catalysts 10-18 are in the optimum range.

### Examples 54-63 and Performance Examples 64-91: Formation of catalysts by direct drying of the slurry

As described below, Catalysts 54-63 were prepared similarly by spray drying the slurry, but varying the iron source, where several commercial available iron oxides and iron nitrate were used to prepare catalyst suitable for n-butene oxidative dehydrogenation to butadiene. The preparation of catalysts in which the iron and zinc wt% in the final catalyst is about 54% and 15%, respectively, was targeted. Catalysts 58-63 were prepared to demonstrate the use of hydrochloric acid as a dispersant to facilitate the reaction of iron oxide with other catalyst components. Catalyst 62 was prepared to demonstrate the effect of the stirring step at 50°C and Catalyst 63 was prepared to demonstrate the effect of the drying method on the catalyst. Performance Examples 64-73 and 74-91 were performed to demonstrate the conversion and selectivity of Catalysts 54-63 and 58-63, respectively, using the method as described for Performance Examples 19-53.

### Example 54: Formation of catalyst by spray drying using yellow iron (III) oxide monohydrate

Yellow iron (III) oxide monohydrate from Strem (39.63 g) was suspended in 280 mL deionized water. To this was added zinc oxide (8.49 g), cobalt (II) carbonate hydrate (2.35 g), magnesium carbonate (0.29 g), and calcium carbonate (0.09 g). The resulting suspension became viscous upon stirring for 3 hours. It was then spray dried at an outlet temperature of 90-98°C. The catalyst precursor was then dried at a temperature of 120°C for 1 hour and calcined at a temperature of 650°C for 10 hours. The resulting catalyst powder was pressed, and then screened, and the 20/50 mesh fraction was used in n-butene oxidative dehydrogenation.

### Example 55: Formation of catalyst by spray drying using iron (III) oxide

Catalyst 55 was prepared using the same procedure described in Example 54, but iron (III) oxide (35.61 g) was used instead of Yellow iron (III) oxide monohydrate from Strem (39.63 g).

### Example 56: Formation of catalyst by spray drying using iron (III) oxide (Goethite, 39.63 g)

Catalyst 56 was prepared using the same procedure described in Example 54, but iron (III) oxide (Goethite, 39.63 g) was used instead of Yellow iron (III) oxide monohydrate from Strem (39.63 g).

### Example 57: Formation of catalyst by spray drying using iron nitrate nonahydrate (90.09 g)

Catalyst 57 was prepared using the same procedure described in Example 54, but iron nitrate nonanhydrate (90.09 g) was used instead of Yellow iron (III) oxide monohydrate from Strem (39.63 g).

| Table 7: Properties of catalysts prepared from different types of iron oxide | | | | | | |
|---|---|---|---|---|---|---|
| Target Formula | Fe ₁Zn _{0.234}Co_{0.0428} Mg _{0.0066} Ca _{0.0021} Oxide | | | | | |
| Expected Composition | % Fe | % Zn | % Co | % Mg | % Ca | % O |
| (wt%) | 54.50 | 14.93 | 2.46 | 0.16 | 0.08 | balance |
| Catalyst Example | % Fe | % Zn | % Co | % Mg | % Ca | Iron Source |
| 54 | 53.46 | 15.09 | 2.71 | 0.25 | 0.12 | Strem Yellow Fe₂O₃ *H₂O |
| 55 | 54.39 | 14.49 | 2.59 | 0.23 | 0.10 | Fe₂O₃ Aldrich |
| 56 | 28.79 | 15.65 | 2.50 | 0.65 | 0.13 | Goethite SiO₂, Al₂O₃ |
| 57 | 54.68 | 14.28 | 2.51 | 0.19 | 0.11 | Iron Nitrate |

All but one attempt (Catalyst Example 56) produced a catalyst with the expected composition as shown in Table 7. Catalyst Example 56 had an unusually low content of iron due to the presence of silica and alumina in the starting iron oxide (Goethite), which was used to prepare this catalyst. The performance of Catalysts 54 through 57 were tested for n-butene oxidative dehydrogenation to butadiene. The results of these performance tests are given in Table 8.

| Table 8: Performance of catalysts prepared from different types of iron oxides in n-butene oxidative dehydrogenation to butadiene. | | | | | | | |
|---|---|---|---|---|---|---|---|
| Performance Example | Catalyst Example | Time (hrs) | RX SPT Temp (°C) | % n-butene Conversion | % O₂ Conversion | % Selectivity to C₄H₆ | Source of Iron |
| 64 | 54 | 13 | 372 | 72.2 | 89.0 | 92.5 | Strem Yellow Fe₂O₃*H₂O |
| 65 | 54 | 20 | 372 | 73.1 | 91.6 | 92.6 | |
| 66 | 54 | 186 | 377 | 67.4 | 94.2 | 90.3 | |
| 67 | 55 | 3 | 323 | 12.5 | 84.9 | 13.2 | Fe₂O₃ Aldrich |
| 68 | 55 | 18 | 323 | 10.2 | 74.9 | 13.2 | |
| 69 | 56 | 1 | 365 | 13.7 | 73.1 | 35.3 | Goethite Si, Al |
| 70 | 56 | 3 | 375 | 12.3 | 64.0 | 36.3 | |
| 71 | 56 | 10 | 375 | 9.3 | 47.6 | 37.8 | |
| 72 | 57 | 13 | 351 | 33.7 | 85.9 | 72.2 | Iron Nitrate |
| 73 | 57 | 90 | 353 | 35.9 | 86.1 | 72.8 | |

Table 8 shows that Catalyst 54 prepared from yellow iron oxide had the maximum n-butene conversion of about 73% at a selectivity to butadiene of 92.6% (Performance Example 65). The next best catalyst is Catalyst 57 prepared with iron nitrate. Catalyst Example 57 had a maximum n-butene conversion of 35.9% at selectivity to butadiene of 72.8% (Performance Example 73).

### Example 58: Formation of catalyst by spray drying and no HCl dispersant

Yellow iron (III) oxide monohydrate from Strem (39.63 g) was suspended in 280 mL deionized water. To this was added zinc oxide (8.49 g), cobalt (II) carbonate hydrate (2.35 g), magnesium carbonate (0.29 g), and calcium carbonate (0.09 g). The resulting suspension became viscous upon stirring for 2 hours at 50°C. It was then spray dried at an outlet temperature of 90-98°C. The catalyst precursor was then dried at a temperature of 120°C for 1 hour and calcined at a temperature of 650°C for 10 hours. The resulting catalyst powder was pressed, and then screened, and the 20/50 mesh fraction was used in n-butene oxidative dehydrogenation.

### Example 59: Formation of catalyst by spray drying and an Fe:Cl ratio of 1:0.17

Yellow iron (III) oxide monohydrate from Strem (39.63 g) was suspended in 280 mL deionized water. To this was added 13 mL of 6 N HCl (approximately 0.17 mole of Cl per mole iron). The mixture was stirred for 1 hour at room temperature. To the resulting mixture was added zinc oxide (8.49 g), cobalt (II) carbonate hydrate (2.35 g), magnesium carbonate (0.29 g), and calcium carbonate (0.09 g). The mixture became viscous almost immediately. The resulting viscous mixture was stirred for 2 hours at 50°C. It was then spray dried at an outlet temperature of 90-98°C. The catalyst precursor was then dried at a temperature of 120°C for 1 hour and calcined at a temperature of 650°C for 10 hours. The resulting catalyst powder was pressed, and then screened, and the 20/50 mesh fraction was used in n-butene oxidative dehydrogenation.

### Example 60: Formation of catalyst by spray drying and an Fe:Cl ratio of 1:0.23

Catalyst 60 was prepared using the same procedure described in Example 59, but 17.3 mL of 6 N HCl (approximately 0.23 mole of Cl per mole iron) was used instead of 13 mL of 6 N HCl (approximately 0.17 mole of Cl per mole iron).

### Example 61: Formation of catalyst by spray drying and an Fe:Cl ratio of 1:0.35

Catalyst 61 was prepared using the same procedure described in Example 59, but 26 mL of 6 N HCl (approximately 0.35 mole of Cl per mole iron) was used instead of 13 mL of 6 N HCl (approximately 0.17 mole of Cl per mole iron).

### Example 62: Formation of catalyst by spray drying: with, after the addition of the zinc and other components to the acid dispersion of iron oxide, no stirring for 2 hours at 50°C and an Fe:Cl ratio of 1:0.23.

Catalyst 62 was prepared using the same procedure described in Example 60, but with no stirring step at 50°C. Specifically, yellow iron (III) oxide monohydrate from Strem (39.63 g) was suspended in 280 mL deionized water. To this was added 17.3 mL of 6 N HCl (approximately 0.23 mole of Cl per mole iron). The mixture was stirred for 1 hour at room temperature. To the resulting mixture was added zinc oxide (8.49 g), cobalt (II) carbonate hydrate (2.35 g), magnesium carbonate (0.29 g), and calcium carbonate (0.09 g). The resulting mixture became viscous almost immediately and was spray dried at an outlet temperature of 90-98°C without further stirring. The catalyst precursor was then dried at a temperature of 120°C for 1 hour and calcined at a temperature of 650°C for 10 hours. The resulting catalyst powder was pressed, and then screened, and the 20/50 mesh fraction was used in n-butene oxidative dehydrogenation.

### Example 63: Formation of catalyst without spray drying and an Fe:Cl ratio of 1:0.23.

Catalyst 63 was prepared using the same procedure described in Example 60, but was not spray dried. Specifically, yellow iron (III) oxide monohydrate from Strem (39.63 g) was suspended in 280 mL deionized water. To this was added 17.3 mL of 6 N HCl (approximately 0.23 mole of Cl per mole iron). The mixture was stirred for 1 hour at room temperature. To the resulting mixture was added zinc oxide (8.49 g), cobalt (II) carbonate hydrate (2.35 g), magnesium carbonate (0.29 g), and calcium carbonate (0.09 g). The resulting mixture became viscous, and was heated to 93°C to remove most of the water. The resulting thick paste was placed in an oven set at 60°C overnight to complete water removal. The catalyst precursor was then dried at a temperature of 120°C for 1 hour and calcined at a temperature of 650°C for 10 hours. The resulting catalyst powder was pressed, and then screened, and the 20/50 mesh fraction was used in n-butene oxidative dehydrogenation.

| Table 9: Performance of Catalyst Examples 40-45 in n-butene oxidative dehydrogenation to butadiene. | | | | | | | |
|---|---|---|---|---|---|---|---|
| Performance Example | Catalyst Example | Time (hrs) | RX SPT Temp (°C) | % C₄H₈ Conversion | % O₂ Conversion | % Selectivity to C₄H₆ | HCl Fe/Cl ratio |
| 74 | 58 | 14 | 365.5 | 64.6 | 86.1 | 91.0 | 1/0.0 |
| 75 | 58 | 22 | 371.0 | 70.0 | 96.2 | 89.9 | |
| 76 | 58 | 135 | 370.0 | 59.7 | 92.0 | 89.1 | |
| 77 | 59 | 4 | 374.3 | 70.7 | 77.5 | 93.1 | 1/0.17 |
| 78 | 59 | 28 | 378.0 | 79.7 | 95.8 | 91.9 | |
| 79 | 59 | 133 | 378.0 | 70.4 | 94.6 | 90.0 | |
| 80 | 60 | 14 | 370.0 | 78.3 | 89.7 | 92.7 | 1/0.23 |
| 81 | 60 | 24 | 371.8 | 81.4 | 95.5 | 92.3 | |
| 82 | 60 | 161 | 372.0 | 74.1 | 94.2 | 90.5 | |
| 83 | 61 | 14 | 377.0 | 79.6 | 93.0 | 93.6 | 1/0.35 |
| 84 | 61 | 22 | 377.0 | 80.3 | 95.8 | 93.1 | |
| 85 | 61 | 179 | 377.5 | 68.6 | 90.9 | 91.9 | |
| 86 | 62 | 14 | 364.0 | 76.9 | 88.5 | 93.0 | 1/0.23 |
| 87 | 62 | 26 | 369.0 | 81.6 | 95.7 | 92.9 | |
| 88 | 62 | 158 | 369.0 | 74.5 | 92.2 | 92.0 | |
| 89 | 63 | 14 | 373.0 | 80.0 | 91.2 | 92.7 | 1/0.23 |
| 90 | 63 | 25 | 374.5 | 81.5 | 95.2 | 92.8 | |
| 91 | 63 | 157 | 379.0 | 70.5 | 90.8 | 91.3 | |

Table 9 shows that adding HCl as a dispersant results in catalysts with enhanced performance in the oxidative dehydrogenation of n-butene to butadiene particularly after an activation period greater than or equal to 10 hrs on stream. Specifically, adding the HCl caused the n-butene conversion to increase as compared to the conversion of 70% observed for Catalyst 58 (Performance Example 75), i.e., the example with no HCl. All the catalysts that employed HCl as a dispersant (catalysts 59-63, performance examples 77-91) had a n-butene conversion of greater than 76% with 14 hours on stream, with majority of the n-butene conversion routinely achieved greater than 79%, and even up to 81.6% of Performance Example 87 at 26 hrs on stream. It is noted that for all of the catalysts, once the oxygen conversion was greater than or equal to 90%, the catalysts achieved n-butene conversion of greater than 79%. It is noted that Performance Example 77 for Catalyst 59 was measured after 4 hrs of time on stream, before the catalyst reached its optimum performance (e.g., where the oxygen conversion has reached at least 85%).

### Examples 92-95 and Performance Examples 96-107: Formation of catalyst by spray drying Catalysts 92 to 95 were prepared in the same manner as catalyst 60, except that the amount of zinc oxide (g) used to prepare each catalyst is as indicated in Table 10:

| Table 10: Composition of Catalysts 42, 74-77 Prepared with a range of zinc content. | | | | |
|---|---|---|---|---|
| Catalyst Example | Amount of Zinc oxide (g) used in catalyst preparation | Wt % Fe | Wt % Zn | Wt % Co |
| 60 | 8.5 | 55.4 | 12.8 | 2.7 |
| 92 | 9.3 | 53.8 | 14.2 | 2.7 |
| 93 | 10.2 | 53.4 | 15.5 | 2.7 |
| 94 | 11.0 | 52.0 | 16.5 | 2.7 |
| 95 | 11.9 | 51.3 | 17.9 | 2.6 |

The properties of catalyst 60, together with the properties of catalyst 92-95 are given in Table 11. The amount of zinc in the catalysts shown in Table 11 ranges from approximately 12 to 18 weight %. The performance of catalyst 60 in n-butene oxidation is compared to the performance of catalyst 92-95. We can conclude from the performance data shown in Table 11 that catalysts having zinc at weight % levels ranging from 12-18, have optimum performance for n-butene oxidation where the initial n-butene conversion exceeds 75% and the initial selectivity to butadiene exceeds 91% by 23 hours on stream in n-butene oxidation. Even after more than 150 hrs of time on stream these catalysts routinely maintain a conversion of n-butene of greater than 70% and a selectivity to butadiene of greater than 90%.

| Table 11 | | | | | | | |
|---|---|---|---|---|---|---|---|
| Performance example | Catalyst Example | Time (h) | RX SPT Temp (°C) | % C₄H₈ Conversion | % O2 Conversion | % Selectivity to C₄H₆ | %Wt Zn |
| 80 | 60 | 14 | 370 | 78.3 | 89.7 | 92.7 | 12.8 |
| 81 | 60 | 24 | 371.8 | 81.4 | 95.5 | 92.3 | |
| 82 | 60 | 161 | 372 | 74.1 | 94.2 | 90.5 | |
| 96 | 92 | 14 | 370 | 80.5 | 92.9 | 93.3 | 14.2 |
| 97 | 92 | 23 | 369.7 | 81.1 | 95.1 | 93 | |
| 98 | 92 | 156 | 369 | 71.7 | 90.2 | 92.3 | |
| 99 | 93 | 15 | 365.3 | 77.5 | 89.7 | 92.7 | 15.5 |
| 100 | 93 | 23 | 367.4 | 80 | 95.1 | 92.8 | |
| 101 | 93 | 155 | 368.5 | 72.6 | 91.8 | 90.9 | |
| 102 | 94 | 14 | 367.3 | 73.1 | 90.6 | 92 | 16.5 |
| 103 | 94 | 23 | 368.6 | 76.5 | 95.1 | 92.1 | |
| 104 | 94 | 155 | 369.5 | 70.6 | 92 | 91.3 | |
| 105 | 95 | 12 | 368 | 76 | 90.1 | 92.3 | 17.9 |
| 106 | 95 | 25 | 370 | 78.3 | 95.8 | 91.9 | |
| 107 | 95 | 154 | 371 | 68.7 | 89.8 | 90.3 | |

Embodiment 1: A method of making a dehydrogenation catalyst, comprising:
   combining a zinc precursor, an iron precursor, a cobalt precursor, a magnesium precursor, optionally a calcium precursor, and optionally an M precursor, in water to form a mixture, wherein M is selected from cobalt (Co), magnesium (Mg), calcium (Ca), silver (Ag), aluminum (Al), cerium (Ce), cesium (Cs), copper (Cu), potassium (K), lanthanum (La), lithium (Li), manganese (Mn), molybdenum (Mo), sodium (Na), nickel (Ni), phosphorus (P), palladium (Pd), platinum (Pt), ruthenium (Ru), silicon (Si), vanadium (V), tungsten (W), yttrium (Y), as well as combinations comprising at least one of the foregoing, wherein the zinc precursor comprises initial zinc and the iron precursor comprises initial iron;
   adding base to the mixture to form a slurry having a pH of 7 to 8.5;
   aging the slurry at a temperature of greater than or equal to 40°C while agitating;
   filtering a precipitate from the aged slurry to collect a catalyst precursor;
   drying and calcining the catalyst precursor to form the catalyst;
   wherein the catalyst has the formula (I)

      FeZnₐCo_{b}Mg_{c}Ca_{d}ClₑM_{f}Oₓ (I)

      wherein the amounts are in mole ratios relative to 1 mole of iron, "a" is 0.07 to 0.7 moles; "b" is 0.01 to 0.20 moles; "c" is less than or equal to 0.40 moles; "d" is less than or equal to 0.40 moles; "e" is less than or equal to 0.10 moles; "f' is less than or equal to 0.20 moles; and "x" is a number depending on the relative amount and valence of the elements different from oxygen in Formula (I), and wherein greater than or equal to 88% of the initial zinc is recovered,
   wherein and the mole ratio of the initial zinc to the initial iron is less than or equal to 0.35.
Embodiment 2: A method of making a dehydrogenation catalyst, comprising:
   combining a zinc precursor, an iron precursor, a cobalt precursor, a magnesium precursor, optionally a calcium precursor, and optionally an M precursor, in water to form a mixture, wherein M is selected from cobalt (Co), magnesium (Mg), calcium (Ca), silver (Ag), aluminum (Al), cerium (Ce), cesium (Cs), copper (Cu), potassium (K), lanthanum (La), lithium (Li), manganese (Mn), molybdenum (Mo), sodium (Na), nickel (Ni), phosphorus (P), palladium (Pd), platinum (Pt), ruthenium (Ru), silicon (Si), vanadium (V), tungsten (W), yttrium (Y), as well as combinations comprising at least one of the foregoing, wherein the zinc precursor comprises initial zinc and the iron precursor comprises initial iron;
   adding base to the mixture to form a slurry having a pH of 7 to 8.5;
   aging the slurry at a temperature of greater than or equal to 40°C while agitating;
   filtering a precipitate from the aged slurry to collect a catalyst precursor;
   drying and calcining the catalyst precursor to form the catalyst;
   wherein the catalyst has the formula (I)

      FeZnₐCo_{b}Mg_{c}Ca_{d}ClₑM_{f}Oₓ (I)

      wherein the amounts are in mole ratios relative to 1 mole of iron, "a" is 0.07 to 0.7 moles; "b" is 0.01 to 0.20 moles; "c" is less than or equal to 0.40 moles; "d" is less than or equal to 0.40 moles; "e" is less than or equal to 0.10 moles; "f' is less than or equal to 0.20 moles; and "x" is a number depending on the relative amount and valence of the elements different from oxygen in Formula (I),
   wherein if the pH is greater than 8.1 then the aging time is greater than or equal to 90 minutes and/or the aging temperature is greater than or equal to 75°C, and wherein the mole ratio of the initial zinc to the initial iron is less than or equal to 0.35.
Embodiment 3: The method of any of Embodiments 1 - 2, wherein the mole ratio of the initial zinc to the initial iron is 0.20-0.35.
Embodiment 4: The method of any of Embodiments 1 - 3, wherein the pH is 7 to 8.1.
Embodiment 5: The method of any of Embodiments 1 - 4, wherein the pH is 7 to 7.8.
Embodiment 5: The method of any of Embodiments 1 - 3, wherein the pH is 8.1 to 8.5.
Embodiment 6: The method of any of Embodiments 1 - 6, wherein greater than or equal to 90% of the initial zinc is recovered.
Embodiment 7: The method of any of Embodiments 1 - 7, wherein the slurry is aged at the temperature of 40°C to 90°C.
Embodiment 8: The method of any of Embodiments 1 - 8, wherein the base comprises sodium hydroxide and/or ammonium hydroxide.
Embodiment 9: The method of any of Embodiments 1 - 9, wherein the base comprises ammonium hydroxide.
Embodiment 10: The method of any of Embodiments 1 - 10, wherein the precipitate is filtered at a filtering temperature of 40°C to 90°C.
Embodiment 11: The method of any of Embodiments 1 - 11, wherein the M comprise cobalt.
Embodiment 12: The method of any of Embodiments 1 - 12, wherein the zinc precursor and the iron precursor are independently selected from oxide precursors, nitrate precursors, carbonate precursors, halide precursors, and combinations comprising at least one of the foregoing precursors.
Embodiment 13: The method of any of Embodiments 1 - 13, wherein the zinc precursor and the iron precursor are independently selected from nitrate nonahydrate, nitrate hexahydrate, oxide monohydrate, carbonate hydrate, chloride, and combinations comprising at least one of the foregoing.
Embodiment 14: The method of any of Embodiments 1 - 14, wherein the iron precursor is iron nitrate nonahydrate, and the zinc precursor is zinc nitrate hexahydrate.
Embodiment 15: The method of any of Embodiments 1 - 15, wherein the zinc precursor and the iron precursor are independently nitrate precursors.
Embodiment 16: A method of making a dehydrogenation catalyst, comprising combining yellow iron (III) oxide monohydrate with water and an acid to form a slurry, agitating the slurry at a temperature of 25°C to 80°C for a period of greater than or equal to 1 hour to form an acid mixture;
   adding, to the acid mixture, a zinc (Zn) precursor, and optionally a cobalt (Co) precursor, optionally a magnesium (Mg) precursor, and optionally MX, to form a slurry, wherein M is selected, calcium (Ca), silver (Ag), aluminum (Al), cerium (Ce), cesium (Cs), copper (Cu), potassium (K), lanthanum (La), lithium (Li), manganese (Mn), molybdenum (Mo), sodium (Na), nickel (Ni), phosphorus (P), palladium (Pd), platinum (Pt), ruthenium (Ru), silicon (Si), vanadium (V), tungsten (W), yttrium (Y), and combinations comprising at least one of the foregoing, and wherein X is an oxide, nitrate, carbonate, halide, or a hydrate thereof;
   drying to form the catalyst precursor; and
   calcining the catalyst precursor to form the catalyst;
   wherein the catalyst has the formula:

   FeZnₐCo_{b}Mg_{c}Ca_{d}ClₑM_{f}Oₓ (I)

   wherein the amounts are in mole ratios relative to 1 mole of iron, "a" is 0.07 to 0.7 moles; "b" is 0.01 to 0.20 moles; "c" is less than or equal to 0.40 moles; "d" is less than or equal to 0.40 moles; "e" is less than or equal to 0.10 moles; "f" is less than or equal to 0.20 moles; and "x" is a number depending on the relative amount and valence of the elements different from oxygen in Formula (I).
Embodiment 17: A method of making a dehydrogenation catalyst, comprising
   combining a zinc precursor, an iron precursor, and optionally an MX, to form a slurry, wherein M is selected from cobalt (Co), magnesium (Mg), calcium (Ca), silver (Ag), aluminum (Al), cerium (Ce), cesium (Cs), copper (Cu), potassium (K), lanthanum (La), lithium (Li), manganese (Mn), molybdenum (Mo), sodium (Na), nickel (Ni), phosphorus (P), palladium (Pd), platinum (Pt), ruthenium (Ru), silicon (Si), vanadium (V), tungsten (W), yttrium (Y), and combinations comprising at least one of the foregoing, and wherein X is an oxide, nitrate, carbonate, halide, or a hydrate thereof;
   wherein combining comprises combining the iron precursor with the acid to form an acid mixture; agitating the acid mixture for a period of greater than or equal to 0.5 hours; and adding the zinc precursor and the MX to the agitated acid mixture; wherein the acid is selected from hydrochloric acid, ferrous chloride, nitric acid, phosphoric acid, zinc chloride, and combinations comprising at least one of the foregoing; agitating the slurry for greater than or equal to 2 hours at a temperature of greater than or equal to 25°C;
   drying the slurry to form the catalyst precursor; and
   calcining the catalyst precursor to form the catalyst;
   wherein the catalyst has the formula (I)

   FeZnₐCo_{b}Mg_{c}Ca_{d}ClₑM_{f}Oₓ (I)

   wherein the amounts are in mole ratios relative to 1 mole of iron, "a" is 0.07 to 0.7 moles; "b" is 0.01 to 0.20 moles; "c" is less than or equal to 0.40 moles; "d" is less than or equal to 0.40 moles; "e" is less than or equal to 0.10 moles; "f" is less than or equal to 0.20 moles; and "x" is a number depending on the relative amount and valence of the elements different from oxygen in Formula (I), (I),
   wherein the zinc precursor comprises initial zinc and the iron precursor comprises initial iron and wherein the mole ratio of the initial zinc to the initial iron is less than or equal to 0.30.
Embodiment 18: The method of Embodiment 17, wherein combining the zinc precursor, the iron precursor, the MX, and acid comprises
   combining the iron precursor with the acid to form an acid mixture;
   agitating the acid mixture for a period of greater than or equal to 0.5 hours; and
   adding the zinc precursor and the MX to the agitated acid mixture;
   wherein the acid is selected from hydrochloric acid, ferrous chloride, nitric acid, phosphoric acid, zinc chloride, and combinations comprising at least one of the foregoing.
Embodiment 19: The method of any of Embodiments 16 - 18, wherein the acid is selected from hydrochloric acid, ferrous chloride, nitric acid, phosphoric acid, zinc chloride, and combinations comprising at least one of the foregoing.
Embodiment 20: The method of any of Embodiments 16 - 19, wherein the acid is hydrochloric acid.
Embodiment 21: The method of any of Embodiments 16 - 20, wherein hydrochloric acid is present such that the iron to chloride ratio is 1:0.00 to 1:0.35.
Embodiment 22: The method of any of Embodiments 16 - 21, wherein the hydrochloric acid is present such that the iron to chloride ratio is 1:(greater than 0.00) to 1:0.23.
Embodiment 23: The method of any of Embodiments 17 - 22, wherein the iron precursor is yellow iron (III) oxide monohydrate.
Embodiment 24: The method of any of Embodiments 17 - 23, further comprising agitating the slurry at a temperature of 25°C to 80°C for a period of greater than or equal to 1 hour, prior to drying.
Embodiment 25: The method of any of Embodiments 16 - 24, wherein the cobalt (Co) precursor is added to acid mixture.
Embodiment 26: The method of any of Embodiments 16 - 25, wherein the zinc precursor is zinc oxide and the iron precursor is iron oxide.
Embodiment 27: The method of any of Embodiments 16 - 26, wherein the drying comprises spray drying.
Embodiment 28: The method of any of Claims 1 - 16, wherein the mole ratio of the initial zinc to the initial iron is 0.25-0.35.
Embodiment 29: The method of any of Claims 1 - 16, wherein the mole ratio of the initial zinc to the initial iron is 0.28-0.32.
Embodiment 30: The method of any of Claims 1 - 29, wherein the mole ratio of the initial zinc to the initial iron is less than or equal to 0.30.
Embodiment 31: The method of any of Claims 1 - 30, further comprising washing the precursor with water, e.g., deionized water.
Embodiment 32: The method of any of Claims 1 - 31, wherein "c" is 0.001 to 0.40 moles.

All ranges disclosed herein are inclusive of the endpoints, and the endpoints are independently combinable with each other (e.g., ranges of "up to 25 wt%, or, more specifically, 5 wt% to 20 wt%", is inclusive of the endpoints and all intermediate values of the ranges of "5 wt% to 25 wt%," etc.). "Combination" is inclusive of blends, mixtures, alloys, reaction products, and the like. Furthermore, the terms "first," "second," and the like, herein do not denote any order, quantity, or importance, but rather are used to denote one element from another. The terms "a" and "an" and "the" herein do not denote a limitation of quantity, and are to be construed to cover both the singular and the plural, unless otherwise indicated herein or clearly contradicted by context. The suffix "(s)" as used herein is intended to include both the singular and the plural of the term that it modifies, thereby including one or more of that term (e.g., the film(s) includes one or more films).

## Claims

1. A method of making a dehydrogenation catalyst, comprising:
combining a zinc precursor, an iron precursor, a cobalt precursor, a magnesium precursor, optionally a calcium precursor, and optionally an M precursor, in water to form a mixture, wherein M is selected from silver (Ag), aluminum (Al), cerium (Ce), cesium (Cs), copper (Cu), potassium (K), lanthanum (La), lithium (Li), manganese (Mn), molybdenum (Mo), sodium (Na), nickel (Ni), phosphorus (P), palladium (Pd), platinum (Pt), ruthenium (Ru), silicon (Si), vanadium (V), tungsten (W), yttrium (Y), as well as combinations comprising at least one of the foregoing, wherein the zinc precursor comprises initial zinc and the iron precursor comprises initial iron;
adding base to the mixture to form a slurry having a pH of 7 to 8.5;
aging the slurry at a temperature of greater than or equal to 40°C while agitating;
filtering a precipitate from the aged slurry to collect a catalyst precursor;
drying and calcining the catalyst precursor to form the catalyst;
wherein the catalyst has the formula (I)
FeZnₐCo_{b}Mg_{c}Ca_{d}ClₑM_{f}Oₓ (I)
wherein the amounts are in mole ratios relative to 1 mole of iron, "a" is 0.07 to 0.7 moles; "b" is 0.01 to 0.20 moles; "c" is 0.001 to 0.40 moles; "d" is less than or equal to 0.40 moles; "e" is less than or equal to 0.10 moles; "f" is less than or equal to 0.20 moles; and "x" is a number depending on the relative amount and valence of the elements different from oxygen in Formula (I), and
one of two following alternatives:
(i) wherein greater than or equal to 88% of the initial zinc is recovered, wherein the mole ratio of the initial zinc to the initial iron is less than or equal to 0.35; or
(ii) wherein if the pH is greater than 8.1 then the aging time is greater than or equal to 90 minutes and/or the aging temperature is greater than or equal to 75°C; and wherein the mole ratio of the initial zinc to the initial iron is less than or equal to 0.35.

2. The method of Claim 1, wherein the mole ratio of the initial zinc to the initial iron is 0.28 to 0.32.

3. The method of Claim 1, wherein the pH is 7 to 8.1.

4. The method of Claim 1, wherein the pH is greater than 8.1 to 8.5.

5. The method of Claim 1, wherein greater than or equal to 90% of the initial zinc is recovered.

6. The method of Claim 1, wherein the zinc precursor and the iron precursor are independently selected from nitrate nonahydrate, nitrate hexahydrate, oxide monohydrate, carbonate hydrate, chloride, and combinations comprising at least one of the foregoing.

7. The method of Claim 1, wherein the iron precursor is iron nitrate nonahydrate, and the zinc precursor is zinc nitrate hexahydrate.

8. A method of making a dehydrogenation catalyst, comprising
combining yellow iron (III) oxide monohydrate with water and an acid to form a slurry;
agitating the slurry at a temperature of 25°C to 80°C for a period of greater than or equal to 1 hour to form an acid mixture;
adding, to the acid mixture, a zinc (Zn) precursor, and a cobalt (Co) precursor, optionally a magnesium (Mg) precursor, optionally a calcium (Ca) precursor, and optionally MX, to form a slurry, wherein M is selected from silver (Ag), aluminum (Al), cerium (Ce), cesium (Cs), copper (Cu), potassium (K), lanthanum (La), lithium (Li), manganese (Mn), molybdenum (Mo), sodium (Na), nickel (Ni), phosphorus (P), palladium (Pd), platinum (Pt), ruthenium (Ru), silicon (Si), vanadium (V), tungsten (W), yttrium (Y), and combinations comprising at least one of the foregoing, and wherein X is an oxide, nitrate, carbonate, halide, or a hydrate thereof;
drying to form a catalyst precursor; and
calcining the catalyst precursor to form the catalyst;
wherein the catalyst has the formula:
FeZnₐCo_{b}Mg_{c}Ca_{d}ClₑM_{f}Oₓ (I)
wherein the amounts are in mole ratios relative to 1 mole of iron, "a" is 0.07 to 0.7 moles; "b" is 0.01 to 0.20 moles; "c" is less than or equal to 0.40 moles; "d" is less than or equal to 0.40 moles; "e" is less than or equal to 0.10 moles; "f" is less than or equal to 0.20 moles; and "x" is a number depending on the relative amount and valence of the elements different from oxygen in Formula (I).

9. A method of making a dehydrogenation catalyst, comprising
combining a zinc precursor, an iron precursor, a cobalt (Co) precursor, optionally a magnesium (Mg) precursor, optionally a calcium (Ca) precursor, and optionally an MX, to form a slurry, wherein M is selected from silver (Ag), aluminum (Al), cerium (Ce), cesium (Cs), copper (Cu), potassium (K), lanthanum (La), lithium (Li), manganese (Mn), molybdenum (Mo), sodium (Na), nickel (Ni), phosphorus (P), palladium (Pd), platinum (Pt), ruthenium (Ru), silicon (Si), vanadium (V), tungsten (W), yttrium (Y), and combinations comprising at least one of the foregoing, and wherein X is an oxide, nitrate, carbonate, halide, or a hydrate thereof;
wherein combining comprises combining the iron precursor with an acid to form an acid mixture; agitating the acid mixture for a period of greater than or equal to 0.5 hours; and adding the zinc precursor and the MX to the agitated acid mixture; wherein the acid is selected from hydrochloric acid, ferrous chloride, nitric acid, phosphoric acid, zinc chloride, and combinations comprising at least one of the foregoing;
agitating the slurry for greater than or equal to 2 hours at a temperature of greater than or equal to 25°C;
drying the slurry to form the catalyst precursor; and
calcining the catalyst precursor to form the catalyst;
wherein the catalyst has the formula (I)
FeZnₐCo_{b}Mg_{c}Ca_{d}ClₑM_{f}Oₓ (I)
wherein the amounts are in mole ratios relative to 1 mole of iron, "a" is 0.07 to 0.7 moles; "b" is 0.01 to 0.20 moles; "c" is less than or equal to 0.40 moles; "d" is less than or equal to 0.40 moles; "e" is less than or equal to 0.10 moles; "f" is less than or equal to 0.20 moles; and "x" is a number depending on the relative amount and valence of the elements different from oxygen in Formula (I),
wherein the zinc precursor comprises initial zinc and the iron precursor comprises initial iron and wherein the mole ratio of the initial zinc to the initial iron is less than or equal to 0.30.

10. The method of Claim 8, wherein the acid is selected from hydrochloric acid, ferrous chloride, nitric acid, phosphoric acid, zinc chloride, and combinations comprising at least one of the foregoing.

11. The method of any of Claims 8 - 9, wherein the hydrochloric acid is present such that the iron to chloride ratio is 1:0.00 to 1:0.35.

12. The method of Claim 9, wherein the iron precursor is yellow iron (III) oxide monohydrate.

13. The method of Claim 8, wherein and the mole ratio of the initial zinc to the initial iron is 0.28 to 0.32.

## Patentansprüche

1. Verfahren zur Herstellung eines Dehydrierungskatalysators, umfassend:
Kombinieren eines Zinkvorläufers, eines Eisenvorläufers, eines Kobaltvorläufers, eines Magnesiumvorläufers, gegebenenfalls eines Calciumvorläufers und gegebenenfalls eines M-Vorläufers, in Wasser, um ein Gemisch zu bilden, wobei M ausgewählt ist aus Silber (Ag), Aluminium (Al), Cer (Ce), Cäsium (Cs), Kupfer (Cu), Kalium (K), Lanthan (La), Lithium (Li), Mangan (Mn), Molybdän (Mo), Natrium (Na), Nickel (Ni), Phosphor (P), Palladium (Pd), Platin (Pt), Ruthenium (Ru), Silizium (Si), Vanadium (V), Wolfram (W), Yttrium (Y) sowie Kombinationen, die mindestens eine der vorgenannten Komponenten umfassen, wobei der Zinkvorläufer Ausgangszink umfasst und der Eisenvorläufer Ausgangseisen umfasst;
Zugabe einer Base zum Gemisch, um eine Aufschlämmung mit einem pH-Wert von 7 bis 8,5 zu bilden;
Altern der Aufschlämmung bei einer Temperatur von mehr als oder gleich 40°C während eines Rührens;
Filtern eines Niederschlags aus der gealterten Aufschlämmung, um einen Katalysatorvorläufer aufzufangen;
Trocknen und Kalzinieren des Katalysatorvorläufers, um den Katalysator zu bilden;
wobei der Katalysator die Formel (I) besitzt:
FeZnₐCo_{b}Mg_{c}Ca_{d}ClₑM_{f}Oₓ (I)
wobei die Mengen in Molverhältnissen relativ zu 1 Mol Eisen vorliegen, "a" 0,07 bis 0,7 Mol ist; "b" 0,01 bis 0,20 Mol ist; "c" 0,001 bis 0,40 Mol ist; "d" kleiner als oder gleich 0,40 Mol ist; "e" kleiner als oder gleich 0,10 Mol ist; "f' kleiner als oder gleich 0,20 Mol ist; und "x" eine Zahl ist, die von der relativen Menge und Wertigkeit der Elemente abhängt, die sich von Sauerstoff in Formel (I) unterscheiden, und
eine von zwei folgenden Alternativen erfüllt ist:
(i) wobei mehr als oder gleich 88% des Ausgangszinks zurückgewonnen werden, wobei das Molverhältnis des Ausgangszinks zum Ausgangseisen kleiner als oder gleich 0,35 ist; oder
(ii) wobei, wenn der pH-Wert größer als 8,1 ist, die Alterungszeit größer als oder gleich 90 Minuten ist und/oder die Alterungstemperatur größer als oder gleich 75°C ist; und wobei das Molverhältnis von Ausgangszink zu Ausgangseisen kleiner als oder gleich 0,35 ist.

2. Verfahren nach Anspruch 1, wobei das Molverhältnis des Ausgangszinks zum Ausgangseisen 0,28 bis 0,32 ist.

3. Verfahren nach Anspruch 1, wobei der pH-Wert 7 bis 8,1 ist.

4. Verfahren nach Anspruch 1, wobei der pH-Wert größer als 8,1 bis 8,5 ist.

5. Verfahren nach Anspruch 1, wobei mehr als oder gleich 90% des Ausgangszinks zurückgewonnen werden.

6. Verfahren nach Anspruch 1, wobei der Zinkvorläufer und der Eisenvorläufer unabhängig voneinander ausgewählt sind aus Nitratnonahydrat, Nitrathexahydrat, Oxidmonohydrat, Carbonathydrat, Chlorid und Kombinationen, die mindestens eine der vorgenannten Komponenten umfassen.

7. Verfahren nach Anspruch 1, wobei der Eisenvorläufer Eisennitrat-Nonahydrat ist und der Zinkvorläufer Zinknitrat-Hexahydrat ist.

8. Verfahren zur Herstellung eines Dehydrierungskatalysators, umfassend Kombinieren von Eisen(III)oxid-Monohydrat mit Wasser und einer Säure, um eine Aufschlämmung zu bilden;
Rühren der Aufschlämmung bei einer Temperatur von 25°C bis 80°C für einen Zeitraum von mehr als oder gleich 1 Stunde, um ein Säuregemisch zu bilden;
Zugabe eines Zink-(Zn)-Vorläufers und eines Kobalt-(Co)-Vorläufers, gegebenenfalls eines Magnesium-(Mg)-Vorläufers, gegebenenfalls eines Calcium-(Ca)-Vorläufers und gegebenenfalls von MX zum Säuregemisch, um eine Aufschlämmung zu bilden, wobei M ausgewählt ist aus Silber (Ag), Aluminium (Al), Cer (Ce), Cäsium (Cs), Kupfer (Cu), Kalium (K), Lanthan (La), Lithium (Li), Mangan (Mn), Molybdän (Mo), Natrium (Na), Nickel (Ni), Phosphor (P), Palladium (Pd), Platin (Pt), Ruthenium (Ru), Silizium (Si), Vanadium (V), Wolfram (W), Yttrium (Y) und Kombinationen, die mindestens eine der vorgenannten Komponenten umfassen, und wobei X ein Oxid, Nitrat, Carbonat, Halogenid oder ein Hydrat davon ist;
Trocknen, um einen Katalysatorvorläufer zu bilden; und
Kalzinieren des Katalysatorvorläufers, um den Katalysator zu bilden;
wobei der Katalysator die Formel besitzt:
FeZnₐCo_{b}Mg_{c}Ca_{d}ClₑM_{f}Oₓ (I)
wobei die Mengen in Molverhältnissen relativ zu 1 Mol Eisen vorliegen, "a" 0,07 bis 0,7 Mol ist, "b" 0,01 bis 0,20 Mol ist, "c" kleiner als oder gleich 0,40 Mol ist, "d" kleiner als oder gleich 0,40 Mol ist, "e" kleiner als oder gleich 0,10 Mol ist, "f kleiner als oder gleich 0,20 Mol ist und "x" eine Zahl ist, die von der relativen Menge und Wertigkeit der Elemente abhängt, die sich von Sauerstoff in Formel (1) unterscheiden.

9. Verfahren zur Herstellung eines Dehydrierungskatalysators, umfassend
Kombinieren eines Zinkvorläufers, eines Eisenvorläufers, eines Kobalt-(Co)-Vorläufers, gegebenenfalls eines Magnesium-(Mg)-Vorläufers, gegebenenfalls eines Calcium-(Ca)-Vorläufers und gegebenenfalls eines MX, um eine Aufschlämmung zu bilden, wobei M ausgewählt ist aus Silber (Ag), Aluminium (Al), Cer (Ce), Cäsium, (Cs), Kupfer (Cu), Kalium (K), Lanthan (La), Lithium (Li), Mangan (Mn), Molybdän (Mo), Natrium (Na), Nickel (Ni), Phosphor (P), Palladium (Pd), Platin (Pt), Ruthenium (Ru), Silizium (Si), Vanadium (V), Wolfram (W), Yttrium (Y) und Kombinationen, die mindestens eine der vorgenannten Komponenten umfassen, und wobei X ein Oxid, Nitrat, Carbonat, Halogenid oder ein Hydrat davon ist;
wobei das Kombinieren das Kombinieren des Eisenvorläufers mit einer Säure, um ein Säuregemisch zu bilden, das Rühren des Säuregemisches für einen Zeitraum von mehr als oder gleich 0,5 Stunden und die Zugabe des Zinkvorläufers und des MX zum gerührten Säuregemisch umfasst, wobei die Säure ausgewählt ist aus Salzsäure, Eisenchlorid, Salpetersäure, Phosphorsäure, Zinkchlorid und Kombinationen, die mindestens eine der vorgenannten Komponenten umfassen;
Rühren der Aufschlämmung für mehr als oder gleich 2 Stunden bei einer Temperatur von mehr als oder gleich 25°C;
Trocknen der Aufschlämmung, um den Katalysatorvorläufer zu bilden; und
Kalzinieren des Katalysatorvorläufers, um den Katalysator zu bilden;
wobei der Katalysator die Formel (I) besitzt:
FeZnₐCo_{b}Mg_{c}Ca_{d}ClₑM_{f}Oₓ (I)
wobei die Mengen in Molverhältnissen relativ zu 1 Mol Eisen vorliegen, "a" 0,07 bis 0,7 Mol beträgt, "b" 0,01 bis 0,20 Mol beträgt, "c" kleiner als oder gleich 0,40 Mol ist, "d" kleiner als oder gleich 0,40 Mol ist, "e" kleiner als oder gleich 0,10 Mol ist, "f kleiner als oder gleich 0,20 Mol ist und "x" eine Zahl ist, die von der relativen Menge und Wertigkeit der Elemente abhängt, die sich von Sauerstoff in Formel (I) unterscheiden,
wobei der Zinkvorläufer Ausgangszink umfasst und der Eisenvorläufer Ausgangseisen umfasst, und wobei das Molverhältnis des Ausgangszinks zum Ausgangseisen kleiner als oder gleich 0,30 ist.

10. Verfahren nach Anspruch 8, wobei die Säure ausgewählt ist aus Salzsäure, Eisenchlorid, Salpetersäure, Phosphorsäure, Zinkchlorid und Kombinationen, die mindestens eine der vorgenannten Komponenten umfassen.

11. Verfahren nach einem der Ansprüche 8 bis 9, wobei die Salzsäure so vorliegt, dass das Eisenchlorid-Verhältnis 1:0,00 bis 1:0,35 beträgt.

12. Verfahren nach Anspruch 9, wobei der Eisenvorläufer Eisen(III)oxid-Monohydrat ist.

13. Verfahren nach Anspruch 8, wobei das Molverhältnis des Ausgangszinks zum Ausgangseisen 0,28 bis 0,32 beträgt.

## Revendications

1. Procédé de fabrication d'un catalyseur de déshydrogénation, comprenant :
la combinaison d'un précurseur de zinc, d'un précurseur de fer, d'un précurseur de cobalt, d'un précurseur de magnésium, éventuellement d'un précurseur de calcium et éventuellement d'un précurseur de M, dans de l'eau pour former un mélange, où M est choisi parmi l'argent (Ag), l'aluminium (Al), le cérium (Ce), le césium (Cs), le cuivre (Cu), le potassium (K), le lanthane (La), le lithium (Li), le manganèse (Mn), le molybdène (Mo), le sodium (Na), le nickel (Ni), le phosphore (P), le palladium (Pd), le platine (Pt), le ruthénium (Ru), le silicium (Si), le vanadium (V), le tungstène (W), l'yttrium (Y), et les combinaisons comprenant au moins l'un des précédents, où le précurseur de zinc comprend du zinc initial et le précurseur de fer comprend du fer initial ;
l'addition d'une base au mélange pour former une suspension ayant un pH de 7 à 8,5 ;
le vieillissement de la suspension à une température supérieure ou égale à 40°C tout en agitant ;
la filtration d'un précipité à partir de la suspension vieillie pour recueillir un précurseur de catalyseur ;
le séchage et la calcination du précurseur de catalyseur pour former le catalyseur ;
où le catalyseur a la formule (I)
FeZnₐCo_{b}Mg_{c}Ca_{d}ClₑM_{f}Oₓ (I)
dans laquelle les quantités sont en rapports molaires par rapport à 1 mole de fer, « a » est de 0,07 à 0,7 mole ; « b » est de 0,01 à 0,20 mole ; « c » est de 0,001 à 0,40 mole ; « d » est inférieur ou égal à 0,40 mole ; « e » est inférieur ou égal à 0,10 mole ; « f » est inférieur ou égal à 0,20 mole ; et « x » est un nombre dépendant de la quantité relative et de la valence des éléments différents de l'oxygène dans la formule (I), et
l'une des deux alternatives :
(i) où un pourcentage supérieur ou égal à 88 % du zinc initial est récupéré, où le rapport molaire du zinc initial sur le fer initial est inférieur ou égal à 0,35 ; ou
(ii) où si le pH est supérieur à 8,1, alors la durée du vieillissement est supérieure ou égale à 90 minutes et/ou la température du vieillissement est supérieure ou égale à 75°C ; et où le rapport molaire du zinc initial sur le fer initial est inférieur ou égal à 0,35.

2. Procédé selon la revendication 1, dans lequel le rapport molaire du zinc initial sur le fer initial est de 0,28 à 0,32.

3. Procédé selon la revendication 1, dans lequel le pH est de 7 à 8,1.

4. Procédé selon la revendication 1, dans lequel le pH est de plus de 8,1 à 8,5.

5. Procédé selon la revendication 1, dans lequel un pourcentage supérieur ou égal à 90 % du zinc initial est récupéré.

6. Procédé selon la revendication 1, dans lequel le précurseur de zinc et le précurseur de fer sont choisis indépendamment parmi le nitrate nonahydraté, le nitrate hexahydraté, l'oxyde monohydraté, le carbonate hydraté, le chlorure, et les combinaisons comprenant au moins l'un des précédents.

7. Procédé selon la revendication 1, dans lequel le précurseur de fer est le nitrate de fer nonahydraté, et le précurseur de zinc est le nitrate de zinc hexahydraté.

8. Procédé de fabrication d'un catalyseur de déshydrogénation, comprenant
la combinaison d'un oxyde de fer (III) jaune monohydraté avec de l'eau et d'un acide pour former une suspension ;
l'agitation de la suspension à une température de 25 °C à 80 °C pendant une durée supérieure ou égale à 1 heure pour former un mélange acide ;
l'addition, au mélange acide, d'un précurseur de zinc (Zn) et d'un précurseur de cobalt (Co), éventuellement d'un précurseur de magnésium (Mg), éventuellement d'un précurseur de calcium (Ca) et éventuellement de MX, pour former une suspension, où M est choisi parmi l'argent (Ag), l'aluminium (Al), le cérium (Ce), le césium (Cs), le cuivre (Cu), le potassium (K), le lanthane (La), le lithium (Li), le manganèse (Mn), le molybdène (Mo), le sodium (Na), le nickel (Ni), le phosphore (P), le palladium (Pd), le platine (Pt), le ruthénium (Ru), le silicium (Si), le vanadium (V), le tungstène (W), l'yttrium (Y), et les combinaisons comprenant au moins l'un des précédents, et où X est un oxyde, un nitrate, un carbonate, un halogénure ou un hydrate de ceux-ci ;
le séchage pour former un précurseur de catalyseur ; et
la calcination du précurseur de catalyseur pour former le catalyseur ;
où le catalyseur a la formule (I)
FeZnₐCO_{b}Mg_{c}Ca_{d}ClₑM_{f}Oₓ (I)
dans laquelle les quantités sont en rapports molaires par rapport à 1 mole de fer, « a » est de 0,07 à 0,7 mole ; « b » est de 0,01 à 0,20 mole ; « c » est inférieur ou égal à 0,40 mole ; « d » est inférieur ou égal à 0,40 mole ; « e » est inférieur ou égal à 0,10 mole ; « f » est inférieur ou égal à 0,20 mole ; et « x » est un nombre dépendant de la quantité relative et de la valence des éléments différents de l'oxygène dans la formule (I).

9. Procédé de fabrication d'un catalyseur de déshydrogénation, comprenant :
la combinaison d'un précurseur de zinc, d'un précurseur de fer, d'un précurseur de cobalt (Co), éventuellement d'un précurseur de magnésium (Mg), éventuellement d'un précurseur de calcium (Ca), et éventuellement de MX, pour former une suspension, où M est choisi parmi l'argent (Ag), l'aluminium (Al), le cérium (Ce), le césium (Cs), le cuivre (Cu), le potassium (K), le lanthane (La), le lithium (Li), le manganèse (Mn), le molybdène (Mo), le sodium (Na), le nickel (Ni), le phosphore (P), le palladium (Pd), le platine (Pt), le ruthénium (Ru), le silicium (Si), le vanadium (V), le tungstène (W), l'yttrium (Y), et les combinaisons comprenant au moins l'un des précédents, où X est un oxyde, un nitrate, un carbonate, un halogénure ou un hydrate de ceux-ci ;
où la combinaison comprend la combinaison du précurseur de fer avec un acide pour former un mélange acide ; l'agitation du mélange acide pendant une durée supérieure ou égale à 0,5 heure ; et l'addition du précurseur de zinc et du MX au mélange d'acide agité ; où l'acide est choisi parmi l'acide chlorhydrique, le chlorure ferreux, l'acide nitrique, l'acide phosphorique, le chlorure de zinc, et les combinaisons comprenant au moins l'un des précédents ;
l'agitation de la suspension pendant une durée supérieure ou égale à 2 heures à une température supérieure ou égale à 25°C ;
le séchage de la suspension pour former le précurseur de catalyseur ; et
la calcination du précurseur de catalyseur pour former le catalyseur ;
où le catalyseur a la formule (I)
FeZnₐCo_{b}Mg_{c}Ca_{d}ClₑM_{f}Oₓ (I)
dans laquelle les quantités sont en rapports molaires par rapport à 1 mole de fer, « a » est de 0,07 à 0,7 mole ; « b » est de 0,01 à 0,20 mole ; « c » est inférieur ou égal à 0,40 mole ; « d » est inférieur ou égal à 0,40 mole ; « e » est inférieur ou égal à 0,10 mole ; « f » est inférieur ou égal à 0,20 mole ; et « x » est un nombre dépendant de la quantité relative et de la valence des éléments différents de l'oxygène dans la formule (I),
où le précurseur de zinc comprend du zinc initial et le précurseur de fer comprend du fer initial et le rapport molaire du zinc initial sur le fer initial est inférieur ou égal à 0,30.

10. Procédé selon la revendication 8, dans lequel l'acide est choisi parmi l'acide chlorhydrique, le chlorure ferreux, l'acide nitrique, l'acide phosphorique, le chlorure de zinc, et les combinaisons comprenant au moins l'un des précédents.

11. Procédé selon l'une quelconque des revendications 8 à 9, dans lequel l'acide chlorhydrique est présent de telle sorte que le rapport fer sur chlorure soit de 1/0,00 à 1/0,35.

12. Procédé selon la revendication 9, dans lequel le précurseur de fer est l'oxyde de fer (III) jaune monohydraté.

13. Procédé selon la revendication 8, dans lequel le rapport molaire du zinc initial sur le fer initial est de 0,28 à 0,32.
